# EUROPÄISCHE PATENTSCHRIFT

(11) **EP 0 823 652 B1**
(45) Veröffentlichungstag und Bekanntmachung des Hinweises auf die Patenterteilung: **03.01.2001**
(21) Anmeldenummer: 97111804.7
(22) Anmeldetag: 11.07.1997
(51) Int. Cl.: G02B 23/24, A61B 1/00

(54) **Endoskopoptik**
Endoscope optics
Systéme optique pour endoscope

(30) Priorität: 07.08.1996 DE 19631840
(43) Veröffentlichungstag der Anmeldung: 11.02.1998
(73) Patentinhaber: OLYMPUS WINTER & IBE GmbH, 22045 Hamburg (DE)
(72) Erfinder: Wulfsberg, Jens Peter, Dr.-Ing., 22949 Ammersbek (DE)
(74) Vertreter: Emmel, Thomas, Dipl.-Biol., Dr.

(56) Entgegenhaltungen:
- EP-A- 0 113 225
- EP-A- 0 301 288
- EP-A- 0 420 057
- US-A- 4 141 362
- US-A- 5 212 595

## Beschreibung

Die Erfindung bezieht sich auf eine starre Endoskopoptik nach dem Oberbegriff des Anspruches 1.

Solche Endoskopoptiken sind entweder fest in einem Endoskop angeordnet oder so ausgebildet, daß sie mit ihrem distalen lang erstreckten Schaftteil in einen entsprechenden Kanal eines Endoskopes einschiebbar sind.

Gattungsgemäße Endoskopoptiken enthalten eine Reihe von optischen Elementen insbesonder Linsen etc, mit denen eine im distalen Bereich der Optik erzeugte Abbildung zum proximalen Ende übertragen und dort betrachtet werden kann. In der Regel ist dazu im distalen Endbereich der Endoskopoptik ein Objektiv angeordnet. Die Übertragung zum proximalen Ende der Endoskopoptik erfolgt mittels eines das Endoskop in Längsrichtung durchsetzenden Bildleiters, der z.B. eine Stablinse sein kann. Es kann sich dabei allerdings auch um eine Faseroptik handeln. Das proximale Betrachtungssystem kann z.B. eine mit der Endoskopoptik verbindbare bzw. darin angeordnete Videokamera oder ein Okular etc. sein.

Endoskopoptiken müssen vor jedem Eingriff sterilisiert werden. Die Sterilisierung erfolgt nach heutigen Anforderungen durch Autoklavieren im Heißdampf bei ca. 140°. Um zu vermeiden, daß Dampf in die Endoskopoptik eindringt und sich dort in Form von Tröpfchen oder Beschlag auf den Linsenoberflächen absetzt, muß die Endoskopoptik so gut wie möglich abgedichtet sein. In der Regel werden daher die Gehäuse solcher Optiken so weit wie möglich einstückig aus Metall hergestellt. In den Bereichen, in denen dies nicht möglich ist, z.B. im Bereich der am distalen und proximalen Ende vorgesehenen Ende Betrachtungsfenster erfolgt eine aufwendige Verklebung bzw. Einlöten der Fenster nach modernster Technik.

Ein Problem besteht darin, daß bei derart abgedichteten Optiken nach ihrer Herstellung keine nachträglichen Änderungen im Inneren mehr vorgenommen werden können. Das bedeutet, daß alle Linsen bereits bei der Herstellung exakt justiert und befestigt sein müssen. Die Herstellung solcher herkömmlicher Endoskopoptiken ist daher relativ aufwendig.

Aus der US-PS 5,056,902 ist eine mit dem proximalen Ende einer Arthroskopoptik verbindbare Fokussiereinrichtung bekannt geworden, die ein dampfdicht abgedichtetes Gehäuse mit einer darin angeordneten verstellbaren Linse aufweist. Die Linsenverstellung erfolgt über ein veränderbares magnetisches Feld. Ob sich derartige magnetische Einstellvorrichtungen auch in der eigentlichen Endoskopoptik vorsehen lassen, erscheint fraglich. Zumindest dürfte der konstruktive Aufwand erheblich sein.

Die US-PS 5,212,595 beschreibt ebenfalls eine nach außen dampfabgedichtete Endoskopoptik mit einer beweglichen optischen Linse.

Zur Verstellung der Linse ist ein von außen betätigbares Stellglied vorgesehen, das durch einen im Gehäuse vorgesehenen, die Optik umgebenden Balg geführt ist. Der Balg ermöglicht eine Betägigung des Stellglieds zur Verstellung der Linse unter abdichtenden Bedingungen. Auch diese Konstruktion ist relativ aufwendig.

Aufgabe der Erfindung ist es daher, eine gut abgedichtete Endoskopoptik zu schaffen, bei der in konstruktiv einfacher Weise mindestens eines der enthaltenen optischen Elemente zur Justierung und/oder auch zur Änderung der optischen Eigenschaften verstellbar ist.

Gelöst wird diese Aufgabe mittels einer Endoskopoptik, die die kennzeichnenden Merkmale der unabhängigen Ansprüche 1 oder 2 aufweist.

Erfindungsgemäß ist danach vorgesehen, daß mindestens eines der in dem dampfabgedichteten Gehäuse der Endoskopoptik vorgesehenen optischen Elemente beweglich ausgebildet ist. Die Verstellung des optischen Elementes z.B. zur Justierung der Endoskopoptik, aber auch z.B. Falls eine Änderung des Blickwinkels oder der Brennweite gewünscht ist, erfolgt auf mechanische Weise. Konkret ist vorgesehen, daß das Gehäuse der Endoskopoptik in Abhängigkeit von der Anzahl der beweglichen optischen Elemente einen oder mehrere verformbare Wandbereiche aufweist, die bei Verformung ein im Inneren des Gehäuses vorgesehenen, an dem beweglichen optischen Element angreifendes Stellglied betätigen.

Der Begriff verformbare Wandbereiche ist weit zu fassen. Er umfaßt z.B. auch verschiebbare Wandbereiche, die zwar selbst nicht verformbar sind, allerdings mit angrenzenden verformbaren Wandbereichen verbunden sind.

Die verformbaren Wandbereiche können in unterschiedlicher Weise ausgebildet sein. Denkbar ist es z.B., das Gehäuse in einem begrenzten Wandbereich extrem dünnwandig nach Art einer eindiückbaren Membran auszubilden. Von innen könnte z.B. gegen diesen Wandbereich unter Federspannung das freie Ende eines Stellhebels anlegen. In dem Fall, daß der Wandbereich eingedrückt würde, könnte bei entsprechender Lagerung und Verbindung mit z.B. einem optischen Element eine Axialverschiebung dieses Elementes in der Endoskopoptik erfolgen. Genauso gut wäre es aber auch möglich, z.B. eine Drehung oder, falls gewünscht, Radialverschiebung zu bewirken. Soll über die vorzugsweise im proximalen Endbereich der Endoskopoptik ausgebildeten verformbaren Wandbereiche ein distales optisches Element betätigt werden, so kann zur entsprechenden Übertragung ohne weiteres auch eine Schub- und Zugstange vorgesehen werden.

Eine weitere Möglichkeit zur Ausbildung eines verformbaren Wandbereiches bestünde darin, einen Teil des Gehäuses in Form eines z.B. nach außen vorstehenden Metallbalges auszubilden. Bei herkömmlicher Form. d.h. mit umlaufenden Rillen, erlaubt ein derartiger Balg durch entsprechende Betätigung seiner äußeren freien Abschlußfläche unterschiedliche Einstellmöglichkeiten. Er läßt sich z.B. gleichmäßig eindrücken. Genausogut kann man die Abschlußfläche nur einseitig belasten und könnte dabei dann z.B. einen inneren starr verbundenen Hebel verschwenken.

Denkbar wäre es allerdings auch, z.B. einen gewendelten Balg vorzusehen, mit dem sich dann z.B. eine Drehbewegung erzeugen ließe.

Bei beiden genannten Ausgestaltungen kann die Betätigung durch direkten Kontakt des verformbaren Wandbereiches mit einem Finger erfolgen. Zur Rückverformung eines eingedrückten Wandbereiches in die Ausgangsposition können z.B. Rückstellfedern vorgesehen werden, die insbesondere bei der ersten Möglichkeit auch eventuelle neue Beulkräfte der Wand kompensieren könnten.

Zusätzlich kann man bei den unterschiedlichen Ausgestaltungen zur Fixierung der eingestellten Position des optischen Elementes ohne größere Probleme eine äußere Feststelleinrichtung, z.B. eine Raste, vorsehen, mit der sich der verformbare Wandbereich in unterschiedlichen Positionen fixieren läßt.

Die Erfindung ist selbstverständlich nicht auf die erwähnten verformbaren Wandbereiche beschränkt, sondern deckt auch nicht genannte Ausgestaltungen ab, solange diese eine zur Verstellung eines optischen Elementes ausreichende Verformung eines Wandbereiches ermöglichen.

Hauptvorteil der Erfindung ist, daß hierbei auf einfache Weise die Übertragung von mechanischen Stellkräften in ein vollkommen abgedichtetes Gehäuse einer Endoskopoptik ermöglicht wird. Es ist damit möglich, z.B. zunächst grob justierte autoklavierbare Endoskopoptiken herzustellen und die Feinjustierung nachträglich vorzunehmen. Genauso gut kann man aber auch autoklavierbare Endoskopoptiken mit verstellbarer Blickrichtung bzw. mit verstellbaren Brennweiten vorsehen.

Im folgenden soll die Erfindung anhand einer Abbildung näher erläutert werden.

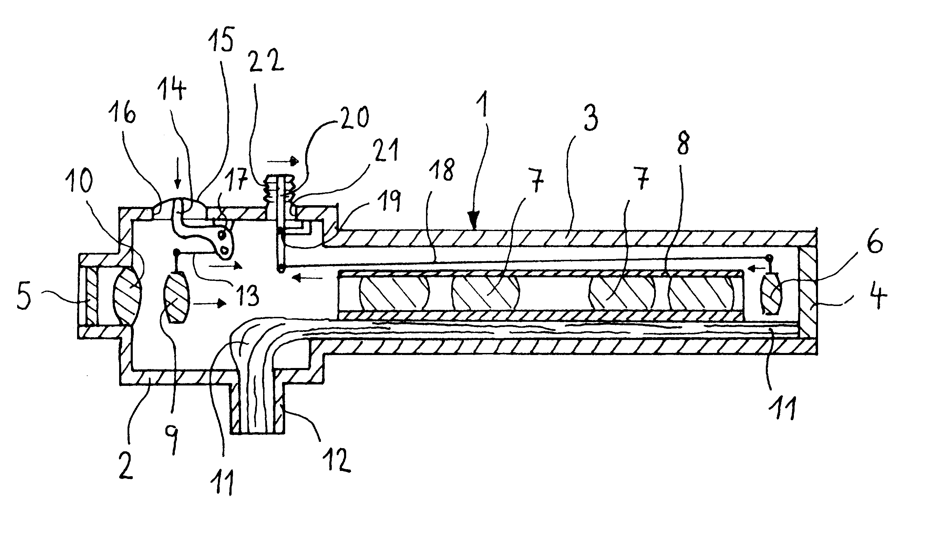

Die Abbildung zeigt eine Endoskopoptik mit einem Gehäuse 1, das einen proximalen Endbereich 2 und einen daran angesetzten distalen Schaft 3 aufweist. Im distalen Ende des Schaftes 3 sowie im proximalen Ende des Gehäusebereiches 2 sind Fenster 4 und 5 vorgesehen. Die Fenster 4 und 5 sind z.B. mittels einer Spezialverlötung so mit dem Gehäuse verbunden, daß eine optimale Abdichtung des Innenraumes des Gehäuses 1 auch bei mehrfacher Autoklavierung gewährleistet ist. Die zur Zeit beste Technik besteht darin, daß die Fenster 4 und 5 an ihrem Außenrand metallisiert sind und während des Einbaus diese Metallschicht mit dem Gehäuse verlötet wird.

Die Endoskopoptik weist als optische Elemente eine im distalen Bereich des Gehäuses angeordnete Objetkivlinse 6, eine der Objektivlinse 6 zugeordnete, mit mehreren langen Stablinsen 7 arbeitende Bildübertragungseinrichtung 8, eine Zoomlinse 9 und eine Okularlinse 10 auf. Zur Beleuchtung des von dem Objektiv 6 abzubildenden Operationsfeldes ist ein Lichtleiter 11 vorgesehen, der von einem proximalen Lichtleiteranschluß 12 bis zu dem distalen Fenster 4 verläuft.

Anhand der Darstellung sollen zwei unterschiedliche Ausführungsformen der erfindungsgemäßen Linsenverstellmechanismen erläutert werden. Die bereits angesprochene Zoomlinse 9 ist in einer nicht dargestellten Schiebeführung in Pfeilrichtung, also in Achsrichtung, verschiebbar gelagert. Die Linse 9 ist an einer Stange 13 angelenkt, die mit dem inneren Ende eines Kniehebels 14 gelenkig verbunden ist. Das andere Ende des Kniehebels 14 liegt gegen eine MeMetallmembran 15 an, die in einer Öffnung 16 des Gehäuseteils 2 eineingelötet ist. Drückt man in Pfeilrichtung auf die Membran 15, so drückt man das obere Ende des Kniehebels 14 nach unten. Der Kniehebel wird dabei um ein Lager 17 verschwenkt und nimmt dabei die Stange 13 in Pfeilrichtung mit, wobei ebenfalls die Zoomlinse verschoben wird.

Eine zweite Ausführungsform betrifft die Verstellung der Objektivlinse 6. Hierzu ist die Linse 6 an einer sich im wesentlichen durch den Schaft 3 des Gehäuses erstreckenden Schub- und Zugstange 18 angelenkt. Die Stange 18 ist mit ihrem proximalen Ende am inneren Ende eines bei 19 im Gehäuseteil 2 gelagerten Hebels 20 gelagert. Ein Metallbalg 22 ist mit seinem unteren innenliegenden Rand an der Offnung 21 verlötet und mit seiner inneren Abschlußfläche mit dem Hebel 20 verbunden. Die Lötverbindung ist so gewählt, daß absolute Dampfdichtigkeit gewährleistet ist. Wird nun der Balg 22 in Pfeilrichtung bewegt, so verschiebt der Hebel 20 die Schub- und Zugstange 18 zum proximalen Ende des Gehäuses 1 und bewegt dabei die in einer nicht gezeigten Schiebeführung gelagerte Objektivlinse 6 entsprechend. Wie bereits oben angesprochen, können außen, also außerhalb der Abdichtung, Einstellschrauben bzw. Rastmechanismen vorgesehen sein, die z.B. auf der Membran 15 oder am Balg 22 anangreifen und eine genaue Einstellung der durch den jeweiligen Mechanismus verstellten Linsen ermöglichen. Zusätzlich können weiterhin Federn vorgesehen sein, die eine Rückstellung der verformbaren Wandbereiche in eine Ausgangsposition automatisch vornehmen. Weiterhin ist selbstverständlich die durch den Balg 22 ermöglichte Bewegung des Hebels 20 nicht auf die dargestellte Verschwenkung beschränkt. Bei entsprechend anderen kooperierenden Stellgliedern wäre es auch ohne weiteres möglich, daß der Hebel unterschiedlich tief in das Gehäuse eingedrückt wird, wie oben bereits angesprochen.

## Patentansprüche

1. Endoskopoptik mit einem starren im wesentlichen schaftförmigen Gehäuse (1), in dem mehrere optische Elemente gegenüber der das Gehäuse umgebenden Atmosphäre dampfabgedichtet aufgenommen sind, wobei mindestens eines der optischen Elemente (6, 9) beweglich ausgebildet ist, und in dem Gehäuse (1) mindestens ein verformbarer Wandbereich (22) vorgesehen ist, der mit einem an dem beweglichen optischen Element (6) angreifenden Stellglied (18) verbunden ist, **dadurch gekennzeichnet,** daß der verformbare Wandbereich (22) als nach außen vorstehender Metallbalg (22) ausgebildet ist und das Stellglied (18) durch Verformung des Wandbereichs (22) betätigbar ist.

2. Endoskopoptik mit einem starren im wesentlichen schaftförmigen Gehäuse (1), in dem mehrere optische Elemente gegenüber der das Gehäuse umgebenden Atmosphäre dampfabgedichtet aufgenommen sind, wobei mindestens eines der optischen Elemente (6, 9) beweglich ausgebildet ist, und in dem Gehäuse (1) mindestens ein verformbarer Wandbereich (15) vorgesehen ist, der mit einem an dem beweglichen optischen Element (9) angreifenden Stellglied (13) bewegungsgekoppelt ist, **dadurch gekennzeichnet,** daß der verformbare Wandbereich (15) als eindrückbare Membran (15) ausgebildet ist und das Stellglied (13) durch Verformung des Wandbereichs (15) betätigbar ist.

## Revendications

1. Optique pour endoscope avec une enveloppe (1) rigide essentiellement en forme de gaine abritant plusieurs éléments optiques de façon à les étanchéifier à la vapeur par rapport à l'atmosphère ambiante, l'un au moins des éléments optiques (6, 9) étant conformé de façon à être mobile et la paroi dans le boîtier (1) comportant au moins une région déformable (22) reliée à un organe de positionnement (18) agissant sur l'élément optique mobile (6), **caractérisé en ce que** la région (22) déformable de la paroi est réalisée sous forme de soufflet métallique faisant saillie vers l'extérieur et en ce que l'organe de positionnement (18) peut être actionné par déformation de la région déformable (22) de la paroi.

2. Optique pour endoscope avec une enveloppe (1) rigide essentiellement en forme de gaine abritant plusieurs éléments optiques de façon à les étanchéifier à la vapeur par rapport à l'atmosphère ambiante, l'un au moins des éléments optiques (6, 9) étant conformé de façon à être mobile et la paroi dans le boîtier (1) comportant au moins une région déformable (15) en accouplement mobile avec un organe de positionnement (13) agissant sur l'élément optique mobile (9), **caractérisé en ce que** la région déformable (15) de la paroi est réalisée sous forme de membrane sur laquelle on peut faire pression de l'extérieur et en ce que l'organe de positionnement (13) peut être actionné par déformation de la région déformable (15) de la paroi.

## Claims

1. Endoscope optical system including a rigid, substantially shaft-shaped housing (1), in which a plurality of optical elements are accommodated in a vapour-sealed manner with respect to the atmosphere surrounding the housing, at least one of the optical elements (6, 9) being constructed to be movable and at least one deformable wall region (22) being provided in the housing (1) which is connected to a control element (18) engaging the movable optical element (6), characterised in that the deformable wall region (22) is constructed in the form of an outwardly projecting metal bellows (22) and the control element (18) is actuable by deformation of the wall region (22).

2. Endoscope optical system with a rigid, substantially shaft-shaped housing (1), in which a plurality of optical elements are accommodated in a vapour-sealed manner with respect to the atmosphere surrounding the housing, at least one of the optical elements (6, 9) being constructed to be movable and at least one deformable wall region (15) being provided in the housing (1) which is movement coupled to a control element (13) engaging the movable optical element (9), characterised in that the deformable wall region (15) is constructed in the form of a pressable membrane (15) and the control element (13) is actuable by deformation of the wall region (15).
